(19) Europäisches Patentamt
European Patent Office
Office européen des brevets

(11) EP 0 402 889 B1

(12) EUROPÄISCHE PATENTSCHRIFT

(45) Veröffentlichungstag und Bekanntmachung des Hinweises auf die Patenterteilung:
**04.09.1996 Patentblatt 1996/36**

(51) Int. Cl.[6]: **C07D 519/00**, C08G 73/06, C08K 5/353
// (C07D519/00, 498:00, 498:00)

(21) Anmeldenummer: **90111176.5**

(22) Anmeldetag: **13.06.1990**

(54) **Polymere Polyalkyl-1-oxa-diazaspirodecane**

Polymeric polyalkyl-1-oxadiazaspirodecanes

Polyalkyl-1-oxadiazaspirodécanes polymères

(84) Benannte Vertragsstaaten:
**AT BE CH DE ES FR GB IT LI NL SE**

(30) Priorität: **16.06.1989 DE 3919691**

(43) Veröffentlichungstag der Anmeldung:
**19.12.1990 Patentblatt 1990/51**

(73) Patentinhaber: **HOECHST AKTIENGESELLSCHAFT**
**65926 Frankfurt am Main (DE)**

(72) Erfinder:
- **Schmailzl, Georg, Dr.**
  **D-8906 Gersthofen (DE)**
- **Pfahler, Gerhard, Dr.**
  **D-8900 Augsburg (DE)**
- **Nowy, Günther, Dr.**
  **D-8901 Aystetten (DE)**

(56) Entgegenhaltungen:
**EP-A- 0 025 867**
**EP-A- 0 028 318**
**EP-A- 0 057 885**
**EP-A- 0 224 181**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 402 889 B1

Printed by Rank Xerox (UK) Business Services
2.13.4/3.4

## Beschreibung

Die Erfindung bezieht sich auf neue Polyalkyl-1-oxa-diazaspirodecane und ihre Verwendung als Lichtschutzmittel für die Stabilisierung von organischen Polymeren gegen Photooxidation.

Hochmolekulare Polyalkylpiperidinstabilisatoren sind bekannt, so beispielsweise das Kondensationsprodukt von N-β-hydroxiethyl-2,2,6,6-tetramethyl-4-hydroxipiperidin mit Bernsteinsäure (vgl. US 4,232,131). Diese Verbindung ist ein Handelsprodukt. Obwohl es bereits sehr gute Wirksamkeit zeigt, kann es dennoch nicht in jeder Hinsicht befriedigen.

Es wurden nunmehr neue, hochwirksame Lichtstabilisatoren gefunden, welche polymere Polyalkyl-1-oxa-diazaspirodecanverbindungen sind.

Die Erfindung betrifft somit polymere Polyalkyl-1-oxa-diazaspirodecane der Formel I

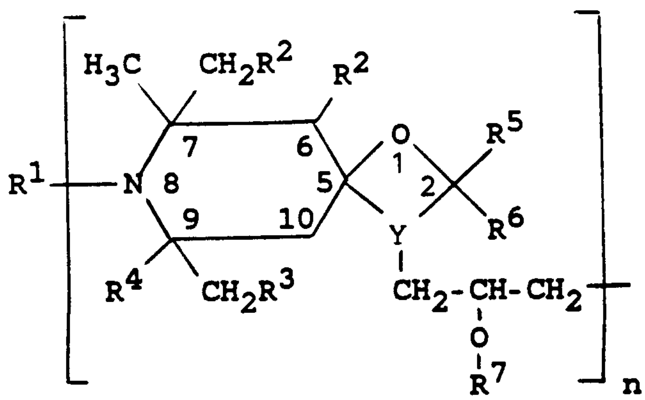

(I),

in welcher

n eine ganze Zahl von 2 bis 50 ist,

Y die Bedeutung einer Gruppe der Formel II oder III hat,

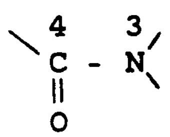

(II)

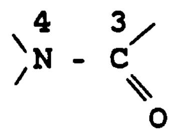

(III)

wobei die Indices 3 bzw. 4 die Ringpositionen im Diazaspirodecansystem angeben und eine Bindung des Stickstoffs mit einer $CH_2$-Gruppe der Propylen-2-oxygruppe verknüpft ist,

$R^1$ ein Wasserstoffatom, ein Sauerstoffatom, eine NO-Gruppe, eine $C_1$-$C_{12}$-Alkylgruppe, eine Allylgruppe, eine $C_1$-$C_{22}$-Acylgruppe oder eine Benzylgruppe bedeutet,

$R^2$ und $R^3$ entweder gleich sind und ein Wasserstoffatom oder eine $C_1$-$C_5$-Alkylgruppe bedeuten, wobei dann

$R^4$ eine Methylgruppe ist, oder

$R^2$ ein Wasserstoffatom oder eine $C_1$-$C_5$-Alkylgruppe ist und

$R^3$ und $R^4$ zusammen mit den sie verbindenden Kohlenstoffatomen eine $C_5$- oder $C_6$-Cycloalkylgruppe oder eine Gruppe der Formel

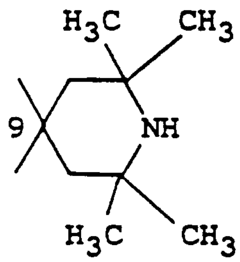

bilden,

$R^5$ und $R^6$ gleich oder verschieden sind und für ein Wasserstoffatom, eine $C_1$-$C_{30}$-Alkylgruppe, für eine unsubstituierte oder durch Chlor oder $C_1$-$C_4$-Alkyl substituierte Phenyl- oder Naphthylgruppe oder für eine unsubstituierte oder durch $C_1$-$C_4$-Alkyl substituierte $C_7$- $C_{12}$-Phenylalkylgruppe stehen, oder

$R^5$ und $R^6$ zusammen mit dem sie verbindenden Kohlenstoffatom eine unsubstituierte oder durch bis zu vier $C_1$-$C_4$-Alkylgruppen substituierte $C_5$-$C_{18}$-Cycloalkylgruppe oder eine Gruppe der Formel

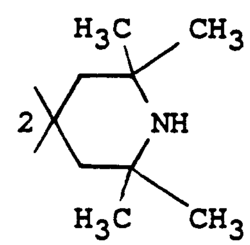

bilden, und

$R^7$ ein Wasserstoffatom oder eine $C_1$-$C_{22}$-Acylgruppe ist oder

$R^7$ in der endständigen Monomereneinheit keine Bedeutung hat, so daß das Sauerstoffatom mit der endständigen $CH_2$-Gruppe verbunden ist und einen Oxiranring bildet.

Die neuen Verbindungen entsprechen der Formel I

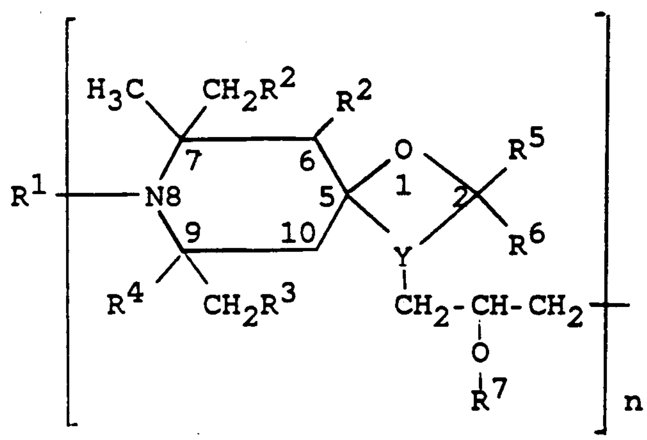 (I),

in welcher

n eine ganze Zahl von 2 bis 50, vorzugsweise von 2 bis 20 und insbesondere von 2 bis 10 ist,

Y hat die Bedeutung einer Gruppe der Formel II oder III

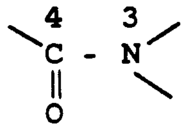 (II) 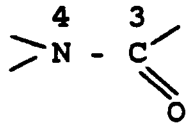 (III),

wobei die Indices 3 bzw. 4 die Ringpositionen im Diazaspirodecansystem angeben und eine Bindung des Stickstoffs mit einer $CH_2$-Gruppe der Propylen-2-oxygruppe verknüpft ist,

$R^1$ ist ein Wasserstoffatom, ein Sauerstoffatom, eine NO-Gruppe, eine $C_1$-$C_{12}$-, vorzugsweise $C_1$-$C_4$-Alkylgruppe, eine Allylgruppe, eine $C_1$-$C_{22}$-Acylgruppe, vorzugsweise Acetyl,oder eine Benzylgruppe. Insbesondere ist $R^1$ ein Wasserstoffatom,

$R^2$ und $R^3$ sind entweder gleich und bedeuten ein Wasserstoffatom oder eine $C_1$-$C_5$-Alkylgruppe, vorzugsweise Wasserstoff, wobei dann

$R^4$ eine Methylgruppe ist, oder

$R^2$ ist ein Wasserstoffatom oder eine $C_1$-$C_5$-Alkylgruppe und

$R^3$ und $R^4$ bilden zusammen mit den sie verbindenden Kohlenstoffatomen eine $C_5$- oder $C_6$-Cycloalkylgruppe oder eine Gruppe der Formel

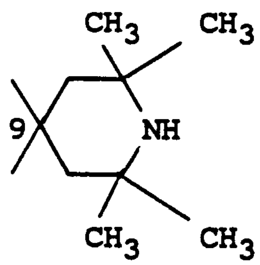

$R^5$ und $R^6$ sind gleich oder verschieden und stehen für ein Wasserstoffatom, eine $C_1$-$C_{30}$-, vorzugswseise $C_1$-$C_{18}$- und insbesondere $C_1$-$C_5$-Alkylgruppe, für eine unsubstituierte oder durch Chlor oder $C_1$-$C_4$-Alkyl substituierte Phenyl- oder Naphthylgruppe, vorzugsweise eine Phenylgruppe, oder für eine unsubstituierte oder durch $C_1$-$C_4$-Alkyl substituierte $C_7$-$C_{12}$-Phenylalkylgruppe, vorzugsweise für eine Benzylgruppe.

Oder

$R^5$ und $R^6$ bilden zusammen mit dem verbindenden Kohlenstoffatom eine unsubstituierte oder durch bis zu vier $C_1$-$C_4$-Alkylgruppen, vorzugsweise Methylgruppen substituierten $C_5$-$C_{18}$, vorzugsweise $C_5$-$C_{12}$-Cycloalkylgruppe oder eine Gruppe der Formel

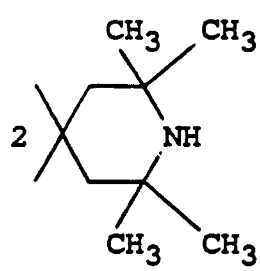

$R^7$ bedeutet ein Wasserstoffatom, eine $C_1$-$C_{22}$-Acylgruppe, vorzugsweise ein Wasserstoffatom oder eine Acetylgruppe und insbesondere ein Wasserstoffatom, oder $R^7$ hat in der endständigen

Monomereneinheit keine Bedeutung, so daß das Sauerstoffatom mit der endständigen $CH_2$-Gruppe verbunden ist und einen Oxiranring bildet.

Beispiele für monomere Ausgangsprodukte (VI), aus welchen die erfindungsgemäßen Oligo- und Polymeren der Formel I gewonnen werden können, sind:

(1) 2,2,7,7,9,9-Hexamethyl-3-(2,3-epoxypropyl)-1-oxa-3,8-diaza-4-oxo-spiro-(4,5)-decan
(2) 2,2,7,7,9,9-Hexamethyl-4-(2,3-epoxypropyl)-1-oxa-3-oxo-4,8-diaza-spiro-(4,5)-decan
(3) 2,2,4,4,10,10,12,12-Octamethyl-7-oxa-3,11,14-triaza-14-(2,3-epoxypropyl)-15-oxo-dispiro-(5,1,5,2)-pentadecan
(4) 2,7,7,9,9-Pentamethyl-2-octadecyl-3-(2,3-epoxypropyl)-1-oxa-3,8-diaza-4-oxo-spiro-(4,5)-decan
(5) 2,7,7,9,9-Pentamethyl-2-benzyl-3-(2,3-epoxypropyl)-1-oxa-3,8-diaza-4-oxo-spiro-(4,5)-decan
(6) 7,7,9,9-Tetramethyl-2,2-diheptyl-3-(2,3-epoxypropyl)-1-oxa-3,8-diaza-4-oxo-spiro-(4,5)-decan
(7) 7,7,9,9-Tetramethyl-2,2-dibenzyl-3-(2,3-epoxypropyl)-1-oxa-3,8-diaza-4-oxo-spiro-(4,5)-decan
(8) 7,7,9,9-Tetramethyl-2-methyl-3-(2,3-epoxypropyl)-1-oxa-3,8-diaza-4-oxo-spiro-(4,5)-decan
(9) 7,7,9,9-Tetramethyl-2-iso-nonyl-3-(2,3-epoxypropyl)-1-oxa-3,8-diaza-4-oxo-spiro-(4,5)-decan
(10) 2,2,4,4-Tetramethyl-7-oxa-3,14-diaza-14-(2,3-epoxypropyl)-15-oxo-dispiro-(5,1,5,2)-pentadecan
(11) 2,2,4,4-Tetramethyl-7-oxa-3,20-diaza-20-(2,3-epoxypropyl)-21-oxo-dispiro-(5,1,11,2)-heneicosan
(12) 2,7,7,9,9-Pentamethyl-2-undecyl-1-oxa-3-oxo-4-(2,3-epoxypropyl)-4,8-diaza-spiro-(4,5)-decan
(13) 7,7,9,9-Tetramethyl-2-ethyl-1-oxa-3-oxo-4-(2,3-epoxypropyl)-4,8-diaza-spiro-(4,5)-decan
(14) 7,7,9,9-Tetramethyl-2-iso-heptyl-1-oxa-3-oxo-4-(2,3-epoxypropyl)-4,8-diaza-spiro-(4,5)-decan
(15) 2,2,4,4-Tetramethyl-7-oxa-3,15-diaza-15-(2,3-epoxypropyl)-14-oxo-dispiro-(5,1,5,2)-pentadecan

sowie Salze dieser Verbindungen mit Protonensäuren.

Die Monomeren (VI) zur Herstellung der neuen Verbindungen werden durch nukleophile Substitution des Halogenatoms im Epihalogenhydrin der Formel V, wobei unter Hal ein Chlor-, Brom- oder Jodatom, bevorzugt Chlor, zu verstehen ist, mit Polyalkyloxadiazaspirodecanen der Formel IV oder deren Salzen mit Protonensäuren gemäß der folgenden Reaktionsgleichung unter Halogenwasserstoffabspaltung erhalten. Anschließendes Erhitzen des Oxirans führt zur Bildung der erfindungsgemäßen Oligo- und Polymeren der Formel I

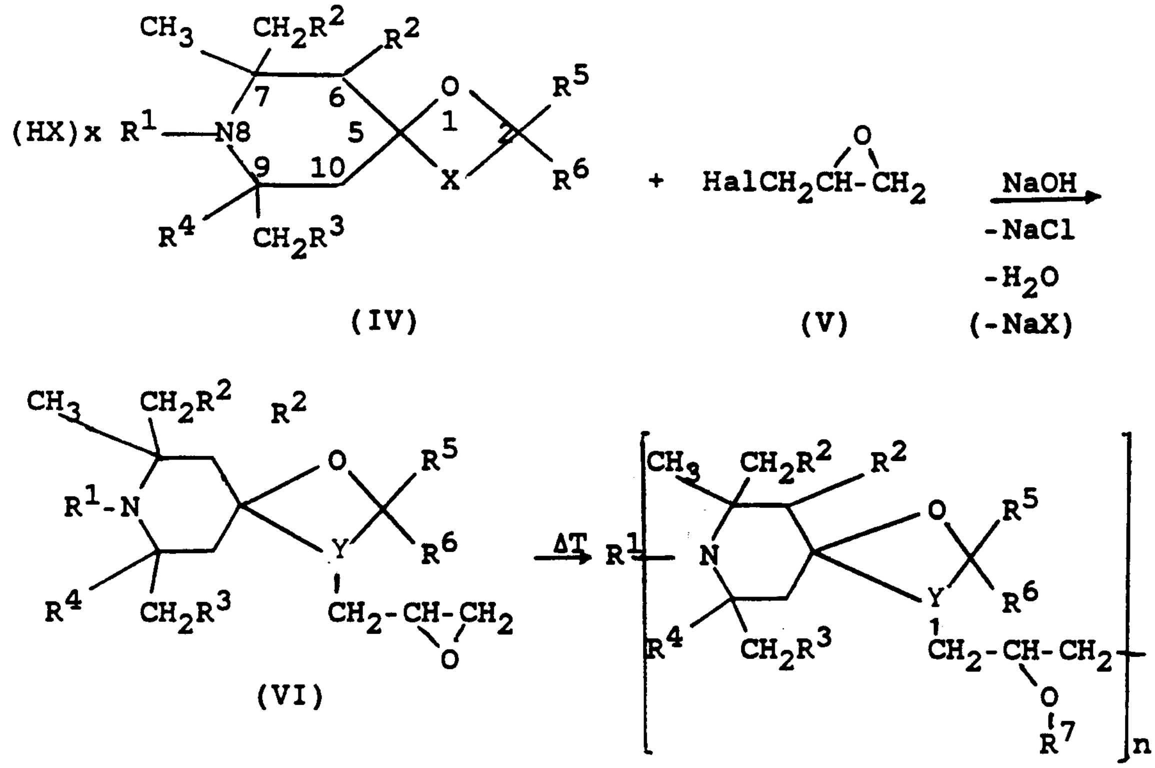

(IV) (V) (VI)

In den Formeln des Reaktionsschemas haben die Reste $R^2$, $R^3$, $R^4$, $R^5$, $R^6$, Y, Hal und n die oben angegebenen Bedeutungen: Der Rest $R^1$ bedeutet Wasserstoff und der Rest $R^7$ bedeutet ebenfalls Wasserstoff oder hat in der endständigen Monomereneinheit keine Bedeutung, so daß das Sauerstoffatom mit der endständigen $CH_2$-Gruppe einen Oxiranring bildet.

Zur Synthese der Verbindungen VI werden die Edukte (IV) und (V) im Molverhältnis 1:1 bis 1:5, vorzugsweise 1:1 bis 1:2 und insbesondere 1:1 bis 1:1,2, in einem inerten organischen Lösemittel in Anwesenheit der äquimolaren bis zwanzigfach molaren Menge festen Alkalimetallhydroxids oder der entsprechenden Menge einer 20- bis 50%igen wäßrigen Lösung eines solchen unter Einsatz eines Phasentransferkatalysators umgesetzt. Die Reaktionstemperatur liegt bei 20 bis 120, vorzugsweise 20 bis 80 und insbesondere 40 bis 60°C.

Als organische Lösemittel kommen aliphatische oder aromatische Kohlenwasserstoffe, wie z.B. Petrolether, Hexan, Heptan, Benzinschnitte, Toluol, Cyclohexan, Xylol etc. in Frage.

Unter Phasentransferkatalysatoren werden Substanzen aus der Gruppe der quartären Ammonium- und Phosphoniumhalogenide verstanden. Auch Polyethylenglykole und Dialkylether von Polyethylenglykolen sind gut geeignet. Man benötigt 0,1 bis 5 Gew.-%, bezogen auf Verbindung (IV).

Die Reaktion ist im allgemeinen nach einer bis 20 Stunden beendet.

Zur Isolierung der Verbindungen VI werden die Phasen, gegebenenfalls nach Zugabe von wenig Wasser, getrennt. Die organische Phase wird mehrfach mit Wasser gewaschen, über einem Trocknungsmittel wie $Na_2SO_4$ oder $MgSO_4$ getrocknet und eingeengt. In den meisten Fällen resultieren ölige Produkte.

Aus den dergestalt erhaltenen Epoxiden lassen sich durch Erhitzen auf 100 bis 240, vorzugsweise 100 bis 200 und insbesondere 120 bis 180°C feste, amorphe, zunächst glasartig anfallende Polymere mit $2 \leq n \leq 50$ gewinnen. Niedrige Polymerisationsgrade können durch kurze und hohe durch lange Polmerisierdauer erzielt werden. Desgleichen wird mit steigender Temperatur die Tendenz zu höheren Polymerisationsgraden beobachtet.

Zur Herstellung der Polymeren oder Oligomeren kann man auch so vorgehen, daß man die Epoxide garnicht erst isoliert, sondern die ganze Reaktionsmischung nach der Umsetzung des Epichlorhydrins mit dem Azaspirodecan auf die genannten höheren Temperaturen bringt und nach erfolgter Polymerisation aufarbeitet.

Nach der Polymerisation kann das Polymer - wenn gewünscht - an der endständigen sekundären Aminfunktion nach an sich bekannten Methoden derivatisiert werden.

Die als Ausgangsprodukte verwendeten Polyalkyloxadiazaspirodecane sind bekannt und nach den in US 4 110 334 und US 4 107 139 angegebenen Vorschriften zugänglich.

Die erfindungsgemäßen Verbindungen dienen als Lichtstabilisatoren in organischen Polymeren, beispielsweise in den nachstehend aufgeführten:

1. Polymere von Mono- und Diolefinen, beispielsweise Polyethylen (das gegebenenfalls vernetzt sein kann), Polypropylen, Polyisobutylen, Polybuten-1, Polymethylpenten-1, Polyisopren oder Polybutadien sowie Polymerisate von Cycloolefinen wie z.B. von Cyclopenten oder Norbornen.

2. Mischungen der unter 1) genannten Polymeren, z.B. Mischungen von Polypropylen mit Polyethylen oder mit Polyisobutylen.

3. Copolymere von Mono- und Diolefinen untereinander oder mit anderen Vinylmonomeren, wie z.B. Ethylen-Propylen-Copolymere, Propylen-Buten-1-Copolymere, Propylen-Isobutylen-Copolymere, Ethylen-Buten-1-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen.

4. Polystyrol.

5. Copolymere von Styrol oder $\alpha$-Methylstyrol mit Dienen oder Acrylderivaten, wie z.B. Styrol-Butadien, Styrol-Maleinsäureanhydrid, Styrol-Acrylnitril, Styrol-Ethylmethacrylat, Styrol-Butadien-Ethylacrylat, Styrol-Acrylnitril-Methylacrylat;
Mischungen von hoher Schlagzähigkeit aus Styrol-Copolymeren und einem anderen Polymer wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Propfcopolymere von Styrol, wie z.B. Styrol auf Polybutadien, Styrol und Acrylnitril auf Polybutadien, Styrol und Maleinsäureanhydrid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie z.B. als sogenannte ABS, MBS, ASA oder AES-Polymere bekannt sind.

7. Halogenhaltige Polymere, wie z.B. Polychloropren, Chlorkautschuk, chloriertes oder chlorsulfoniertes Polyethylen, Epichlorhydrin-homo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere wie Vinylchlorid-Vinylidenchlorid, Vinylchlorid-Vinylacetat oder Vinylidenchlorid-Vinylacetat.

8. Polymere, die sich von $\alpha,\beta$-ungesättigten Säuren und deren Derivaten ableiten, wie Polyacrylate und Polymethacrylate, Polyacrylamide und Polyacrylnitrile.

9. Copolymere der unter 8) genannen Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin.

11. Homo- und Copolymere von cyclischen Ethern, wie Polyethylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere wie z.B. Ethylenoxyd enthalten.

13. Polyphenylenoxyde und -sulfide.

14. Polyurethane, die sich von Polyethern, Polyestern und Polybutadienen mit endständigen Hydroxylgruppen einerseits und aliphatischen oder aromatischen Polyisocyanaten andererseits ableiten sowie deren Vorprodukte (Polyisocyanate-Polyole-Prepolymere).

15. Polyamide und Copolyamide, die sich von Diaminen und Dicarbonsäuren und/oder von Aminocarbonsäuren oder den entsprechenden Lactamen ableiten, wie Polyamid-4, Polyamid-6, Polyamid-6/6, Polyamid-6/10, Polyamid-11, Polyamid-12, Poly-2,4,4-trimethylhexamethylenterephthalamid, Poly-m-phenylen-isophthalamid, sowie deren Copolymere mit Polyethern wie z.B. mit Polyethylenglykol, Polypropylenglykol oder Polytetramethylenglykol.

16. Polyharnstoffe, Polyimide und Polyamid-imide.

17. Polyester, die sich von Dicarbonsäuren und Diolen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Poly-(2,2-bis(4-hydroxyphenyl)-propan-) terephthalat, Polyhydroxybenzoate, sowie Block-Polyetherester, die sich von Polyethylen mit Hydroxyendgruppen, Dialkoholen und Dicarbonsäuren ableiten.

18. Polycarbonate.

19. Polysulfone und Polyethersulfone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten wie z.B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten oder Epoxidharzen vernetzt sind.

25. Vernetzte Epoxidharze, die sich von Polyepoxiden ableiten, z.B. von Bis-glycidylethern oder von cycloaliphatischen Diepoxiden.

26. Natürliche Polymere, wie Cellulose, Naturkautschuk, Gelatine sowie deren polymerhomolog chemisch abgewandelte Derivate, wie Celluloseacetate, -propionate und -butyrate, bzw. die Celluloseether, wie Methylcellulose.

27. Mischungen der oben erwähnten Polymeren, wie beispielsweise PP/EPDM, Polyamid-6/EPDM oder ABS, PVC/EVA, PVC/ABS, PVC/MBS, PC/ABS, PBTP/ABS, PC/ASA, PC/PBT, PVC/CPE, PVD/Acrylat, POM/thermoplastisches PUR, POM/Acrylat, POM/MBS, PPE/HIPS, PPE/Polyamid-6.6 und Copolymere, PA/HDPE, PA/PP, PA/PPE.

28. Natürlich vorkommende und synthetische organische Stoffe, welche reine Monomere oder Mischungen von Monomeren sind, wie beispielsweise Mineralöle, tierische und pflanzliche Fette, Öle und Wachse, oder Öle, Fette und Wachse auf Basis synthetischer Ester oder Mischungen dieser Stoffe.

29. Wäßrige Dispersionen von Natur- oder Synthesekautschuk.

Die neuen Stabilisatoren werden nach allgemein üblichen Methoden in organische Polymere eingearbeitet. Die Einarbeitung kann beispielsweise durch Einmischen der Verbindungen und gegebenenfalls weiterer Additive in die Schmelze vor oder während der Formgebung erfolgen. Auch durch Aufbringen der gelösten oder dispergierten Verbindungen auf das Polymere direkt oder Einmischen in eine Lösung, Suspension oder Emulsion des Polymeren, gegebenenfalls unter nachträglichem Verdunstenlassen des Lösemittels kann die Einarbeitung erfolgen. Die den Polymeren zuzusetzende Menge liegt bei 0,01 bis 10, vorzugsweise 0,05 bis 5, insbesondere 0,1 bis 1,0 Gew.-%, bezogen auf das zu stabilisierende Material.

Die neuen Verbindungen können auch in Form eines Masterbatches, der diese Verbindungen beispielsweise in einer Konzentration von 2,5 bis 50, vorzugsweise 5,0 bis 20 Gew.-% enthält, den zu stabilisierenden Polymeren zugesetzt werden.

Zusätzlich können die zu stabilisierenden organischen Polymeren noch folgende Antioxidatien enthalten, wie beispielsweise:

1. **Alkylierte Monophenole,** beispielsweise
2,6-Di-t-butyl-4-methylphenol,
2-t-Butyl-4,6-dimethylphenol,
2,6-Di-t-butyl-4-ethylphenol,
2,6-Di-t-butyl-4-n-butylphenol,
2,6-Di-t-butyl-4-i-butylphenol,
2,6-Di-cyclopentyl-4-methylphenol,
2-($\alpha$-Methylcyclohexyl)-4,6-dimethylphenol,
2,6-Di-octadecyl-4-methylphenol,
2,4,6-Tri-cyclohexylphenol,
2,6-Di-t-butyl-4-methoxymethylphenol.

2. **Alkylierte Hydrochinone,** beispielsweise
2,6-Di-t-butyl-4-methoxyphenol,
2,5-Di-t-butyl-hydrochinon,
2,5-Di-t-amyl-hydrochinon,
2,6-Diphenyl-4-octadecyloxyphenol.

3. **Hydroxylierte Thiodiphenylether,** beispielsweise
2,2′-Thio-bis-(6-t-butyl-4-methylphenol),
2,2′-Thio-bis(4-octylphenol),
4,4′-Thio-bis-(6-t-butyl-3-methylphenol),
4,4′-Thio-bis-(6-t-butyl-2-methylphenol).

4. **Alkyliden-Bisphenole,** beispielsweise
2,2′-Methylen-bis-(6-t-butyl-4-methylphenol),

2,2′Methylen-bis-(6-t-butyl-4-ethylphenol),
2,2′-Methylen-bis-[4-methyl-6-(α-methylcyclohexyl)-phenol],
2,2′-Methylen-bis-(4-methyl-6-cyclohexylphenol),
2,2′-Methylen-bis-(6-nonyl-4-methylphenol),
2,2′-Methylen-bis- (4,6-di-t-butylphenol),
2,2′-Ethyliden-bis-(4,6-di-t-butylphenol),
2,2′-Ethyliden-bis-(6-t-butyl-4-isobutylphenol),
2,2′-Methylen-bis-[6-(α-methylbenzyl)-4-nonylphenol]
2,2′-Methylen-bis-[6-(α,α-dimethylbenzyl)-4-nonylphenol],
4,4′-Methylen-bis-(2,6-di-t-butylphenol),
4,4′-Methylen-bis(6-t-butyl-2-methylphenol),
1,1-Bis-(5-t-butyl-4-hydroxy-2-methylphenyl)-butan,
2,6-Di-(3-t-butyl-5-methyl-2-hydroxybenzyl)-4-methylphenol,
1,1,3-Tris-(5-t-butyl-4-hydroxy-2-methylphenyl)-butan,
1,1-Bis-(5-t-butyl-4-hydroxy-2-methylphenyl)-3-n-dodecylmercaptobutan,
Di-(3-t-butyl-4-hydroxy-5-methylphenyl)-dicyclopentadien,
Di-[2-(3′-t-butyl-2′-hydroxy-5′-methyl-benzyl)-6-t-butyl-4-methyl-phenyl]-terephthalat,
Ethylenglykol-bis-[3,3-bis(3′-t-butyl-4′-hydroxyphenyl)-butyrat].

5. **Benzylverbindungen,** beispielsweise
1,3,5-Tri-(3,5-di-t-butyl-4-hydroxybenzyl)-2,4,6-tri-methylbenzol,
Di-(3,5-di-t-butyl-4-hydroxybenzyl)-sulfid,
3,5-Di-t-butyl-4-hydroxybenzyl-mercaptoessigsäure-isooctylester,
Bis-(4-t-butyl-3-hydroxy-2,6-dimethylbenzyl)-dithiol-terephthalat,
1,3,5-Tris-(3,5-di-t-butyl-4-hydroxybenzyl)-isocyanurat,
1,3,5-Tris-(4-t-butyl-3-hydroxy-2,6-dimethylbenzyl)-isocyanurat,
3,5-Di-t-butyl-4-hydroxybenzyl-phosphonsäure-dioctadecylester,
Calciumsalz des 3,5-Di-t-butyl-4-hydroxybenzyl-phosphonsäure-mono-ethylesters.

6. **Acylaminophenole,** beispielsweise
4-Hydroxy-laurinsäureanilid,
4-Hydroxy-stearinsäureanilid,
2,4-Bis-octylmercapto-6-(3,5-di-t-butyl-4-hydroxyanilino)-s-triazin,
N-(3,5-di-t-butyl-4-hydroxyphenyl)-carbaminsäure-octylester.

7. Ester der β-(3,5-Di-t-butyl-4-hydroxyphenyl)-propionsäure mit ein- oder mehrwertigen Alkoholen, wie z.B. mit
Methanol,
Octadecanol,
1,6-Hexandiol,
Neopentylglykol,
Thiodiethylenglykol,
Diethylenglykol,
Triethylenglykol,
Pentaerythrit,
Tris-hydroxyethyl-isocyanurat,
Di-hydroxyethyl-oxalsäurediamid.

8. **Ester der β-(5-t-butyl-4-hydroxy-3-methylphenyl)-propionsaure** mit ein- oder mehrwertigen Alkoholen, wie z.B. mit
Methanol,
Octadecanol,
1,6-Hexandiol,
Neopentylglykol,
Thiodiethylenglykol,
Diethylenglykol,
Triethylenglykol,
Pentaerythrit,
Tris-hydroxyethyl-isocyanurat,
Di-hydroxyethyl-oxalsäurediamid.

9. **Amide der β-(3,5-Di-t-butyl-4-hydroxyphenyl)-propionsäure,** wie z.B.
N,N′-Di-(3,5-di-t-butyl-4-hydroxyphenylpropionyl)-hexamethylendiamin,
N,N′-Di-(3,5-di-t-butyl-4-hydroxy-phenylpropionyl)-trimethylendiamin,
N,N′-Di-(3,5-di-t-butyl-4-hydroxyphenylpropionyl)-hydrazin.

Daneben können die zu stabilisierenden Polymeren noch weitere Additive enthalten, wie beispielsweise:

1. UV-Adsorber und Lichtschutzmittel

1.1 **2-(2′-Hydroxyphenyl)-benztriazole,** wie z.B. das 5′-Methyl-, 3′,5′-Di-t-butyl-, 5′-t-Butyl-, 5′-(1,1,3,3-Tetramethylbutyl)-, 5-Chlor-3′,5′-di-t-butyl-, 5-Chlor-3′-t-butyl-5′-methyl-, 3′-sec.-Butyl-5′-t-butyl-, 4′-Octoxy-, 3′,5′-Di-t-amyl-, 3′,5′-Bis-(α,α-dimethylbenzyl)-Derivat.

1.2 **2-Hydroxybenzophenone,** beispielsweise das 4-Hydroxy-, 4-Methoxy-, 4-Octoxy-, 4-Decyloxy-, 4-Dodecyloxy-, 4-Benzyloxy-, 4,2′,4′-Trihydroxy-, 2′-Hydroxy-4,4′-dimethoxy-Derivat.

1.3 **Ester von gegebenenfalls substituierten Benzoesäuren,** beispielsweise
4-t-Butyl-phenylsalicylat, Phenylsalicylat, Octylphenylsalicyalat, Dibenzoylresorcin, Bis-(4-t-butylbenzoyl)-resorcin, Benzoylresorcin, 3,5-Di-t-butyl-4-hydroxy-benzoesäure-2,4-di-t-butylphenylester, 3,5-Di-t-butyl-4-hydroxybenzoesäurehexadecylester.

1.4 **Acrylate,** beispielsweise
α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäuremethylester, N-(β-Carbomethoxy-β-cyano-vinyl)-2-methyl-indolin.

1.5 **Nickelverbindungen,** beispielsweise
Nickelkomplexe des 2,2′-Thio-bis-[4-(1,1,3,3-tetramethyl-butyl)-phenols], wie der 1:1- oder 1:2-Komplex, gegebenenfalls mit zusätzlichen Liganden wie n-Butylamin, Triethanolamin oder N-Cyclohexyl-diethanolamin, Nikkelalkyl-dithiocarbamate, Nickelsalze von 4-Hydroxy-3,5-di-t-butyl-benzylphosphonsäure-monoalkylestern wie vom Methyl- oder Ethylester, Nickelkomplexe von Ketoximen wie von 2-Hydroxy-4-methyl-phenyl-undecylketonoxim, Nickelkomplexe des 1-Phenyl-4-lauroyl-5-hydroxy-pyrazols, gegebenenfalls mit zusätzlichen Liganden, Nickelsalze der 2-Hydroxy-4-alkoxybenzophenone.

1.6 **Sterisch gehinderte Amine,** beispielsweise

1.6.1. Bis-(2,2,6,6-tetramethylpiperidyl)-sebacat, Bis-(1,2,2,6,6-pentamethylpiperidyl)-sebacat, Bis-(2,2,6,6-tetramethylpiperidyl)-glutarat, Bis-(1,2,2,6,6-pentamethylpiperidyl)-glutarat, Bis-(2,2,6,6-tetramethylpiperidyl)-succinat, Bis-(1,2,2,6,6-pentamethylpiperidyl)-succinat, 4-Stearyloxy-2,2,6,6-tetramethylpiperidin, 4-Stearyloxy-1,2,2,6,6-pentamethylpiperidin, 4-Stearoyloxy-2,2,6,6-tetramethylpiperidin, 4-Stearoyloxy-1,2,2,6,6-pentamethylpiperidin, 2,2,6,6-Tetramethylpiperidylbehenat, 1,2,2,6,6-Pentamethylpiperidylbehenat, 2,2,4,4-Tetramethyl-7-oxa-3,20-diazadispiro[5.1.11.2]-heneicosan-21-on, 2,2,3,4,4-Penta-methyl-7-oxa-3,20-diaza-dispiro-[5.1.11.2]-heneicosan-21-on, 2,2,4,4-Tetramethyl-3-acetyl-7oxa-3,20-diaza-dispiro-[5.1.11.2]-heneicosan-21-on, 2,2,4,4-Tetramethyl-7-oxa-3,20-diaza-20-(β-lauryl-oxycarbonylethyl)-21-oxo-dispiro-[5.1.11.2]-heneicosan, 2,2,3,4,4-Pentamethyl-7-oxa-3,20-diaza-20-(β-lauryloxycarbonylethyl)-21-oxo-dispiro-[5.1.11.2.]-heneicosan, 2,2,4,4-Tetramethyl-3-acetyl-7-oxa-3,20-diaza-20-(β-lauryloxycarbonylethyl)-21-oxo-dispiro-[5.1.11.2]-heneicosan, 1,1′,3,3′,5,5′-Hexahydro-2,2′,4,4′,6,6′-hexaaza-2,2′,6,6′-bismethano-7,8-dioxo-4,4′-bis-(1,2,2,6,6-pentamethyl-4-piperidyl)-biphenyl, NN′N″N‴-tetrakis-{2,4-bis-[N-(2,2,6,6-tetramethyl-4-piperidyl)-butylamino]-1,3,5-triazin-6-yl}-4,7-diazadecan-1,10-diamin, NN′N″N‴-tetrakis-{2,4-bis-[N-(1,2,2,6,6-pentamethyl-4-piperidyl)-butylamino]-1,3,5-triazin-6-yl}-4,7-diazadecan-1,10-diamin, NN′N″N‴-tetrakis-{2,4-bis-[N-(2,2,6,6-tetramethyl-4-piperidyl)-methoxypropylamino]-1,3,5-triazin-6-yl}-4,7-diazadecan-1,10-diamin, NN′N″N‴-tetrakis-{2,4-bis-[N-(1,2,2,6,6-pentamethyl-4-piperidyl)-methoxypropylamino]-1,3,5-triazin-6-yl}-4,7-diazadecan-1,10-diamin, Bis-(1,2,2,6,6-pentamethyl-piperidyl)-n-butyl-3,5-di-tert.-butyl-4-hydroxy-benzylmalonat, Tris-(2,2,6,6-tetramethyl-4-piperidyl)-nitrilotriacetat, Tetrakis-(2,2,6,6-tetramethyl-4-piperidyl)-1,2,3,4-butantetracarbonsäure, 1,1′-(1,2-Ethandiyl)-bis-(3,3,5,5-tetramethyl-piperazinon).

1.6.2. Poly-N,N′-bis-(2,2,6,6-tetramethyl-4-piperidyl)-1,8-diazadecylen, Kondensationsprodukt aus 1-(2-Hydroxyethyl)-2,2,6,6-tetramethyl-4-hydroxy-piperidin und Bernsteinsäure, Kondensationsprodukt aus

N,N′-bis-(2,2,6,6-tetramethyl-4-piperidyl)-hexamethylendiamin und 4-tert.-Octylamino-2,6-dichlor-1,3,5-triazin, Kondensationsprodukt aus N,N′-bis-(2,2,6,6-tetramethyl-4-piperidyl)-hexamethylendiamin und 4-Morpholino-2,6-dichlor-1,3,5-triazin.

Besonders vorteilhaft erweist sich dabei in vielen Fällen eine Kombination der erfindungsgemäßen Verbindungen mit den unter 1.6.1 genannten Verbindungen.

1.7 **Oxalsäurediamide,** beispielsweise
4,4′-Di-octyloxy-oxanilid, 2,2′-Di-octyloxy-5,5′-di-t-butyl-oxanilid, 2,2′-Didodecyloxy-5,5′-di-t-butyloxanilid, 2-Ethoxy-2′-ethyl-oxanilid, N,N′-Bis-(3-dimethylaminopropyl)-oxalamid, 2-Ethoxy-5-t-butyl-2′-ethyloxanilid und dessen Gemisch mit 2-Ethoxy-2′-ethyl-5,4-di-t-butyl-oxanilid, Gemische von ortho- und para-Methoxy- sowie von o- und p-Ethoxy-di-substituierten Oxaniliden.

2. **Metalldesaktivatoren**, beispielsweise
N,N′-Diphenyloxalsäurediamid, N-Salicylal-N′-salicyloyl-hydrazin, N,N′-Bis-salicyloyl-hydrazin, N,N′-Bis-(3,5-di-t-butyl-4-hydroxyphenylpropionyl)-hydrazin, 3-Salicyloyl-amino-1,2,3-triazol, Bis-benzylidenoxalsäuredihydrazid.

3. **Phosphite und Phosphonite,** beispielsweise
Triphenylphosphit, Diphenylalkylphosphite, Phenyldialkylphosphite, Trisnonylphenylphosphit, Trilaurylphosphit, Trioctadecylphosphit, Distearyl-pentaerythrityldiphosphit, Tris(2,4-di-t-butylphenyl)phosphit, Diisodecyl-pentaerythrityl-diphosphit, Bis(2,4-di-t-butylphenyl)-pentaerythrityl-diphosphit, Tristearyl-sorbityltriphosphit, Tetrakis-(2,4-di-t-butylphenyl)-4,4′-biphenylen-diphosphonit, 3,9-Bis-(2,4-di-t-butylphenoxy)-2,4,8,10-tetraoxa-3,9-diphosphaspiro(5.5)-undecan, Tris(2-t-butyl-4-thio(2′-methenyl-4′-hydroxy-5′-t-butyl)-phenyl-5-methenyl)-phenylphosphit.

4. **Peroxidzerstörende Verbindungen,** beispielsweise
Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-alkyl-dithiocarbamate, Dioctadecylsulfid, Pentaerythrit-tetrakis-(β-dodecyl-mercapto)-propionat.

5. **Basische Co-Stabilisatoren,** beispielsweise
Melamin, Polyvinylpyrrolidon, Dicyandiamid, Triallylcyanurat, Harnstoff-Derivate, Hydrazin-Derivate, Amine, Polyamine, Polyurethane, Alkali- und Erdalkalisalze höherer Fettsäuren oder Phenolate, beispielsweise Ca-Stearat, Zn-Stearat, Mg-Stearat, Na-Ricinoleat, K-Palmitat, Antimonbrenzcatechinat oder Zinnbrenzcatechinat, Hydroxide und Oxide von Erdalkalimetallen oder des Aluminiums, beispielsweise CaO, MgO, ZnO.

6. Nukleierungsmittel, beispielsweise
4-t-Butylbenzoesäure, Adipinsäure, Diphenylessigsäure, Dibenzylidensorbitol.

7. **Füllstoffe und Verstärkungsmittel,** beispielsweise
Calciumcarbonat, Silikate, Glasfasern, Asbest, Talk, Kaolin, Glimmer, Bariumsulfat, Metalloxide und -hydroxide, Ruß, Graphit.

8. **Sonstige Zusätze,** beispielsweise
Weichmacher, Gleitmittel, Emulgatoren, Pigmente, optische Aufheller, Flammschutzmittel, Antistatika, Treibmittel.

Die verschiedenen zusätzlichen Additive der vorgenannten Gruppen 1 bis 6 werden den zu stabilisierenden Polymeren in einer Menge von 0,01 bis 10, vorzugsweise 0,01 bis 5 Gew.-%, bezogen auf das Gesamtgewicht der Formmasse, zugesetzt. Der Mengenanteil der Additive der Gruppen 7 und 8 beträgt 1 bis 80, vorzugsweise 10 bis 50 Gew.-%, bezogen auf die gesamte Formmasse.

Die erfindungsgemäß stabilisierten organischen Polymeren können in verschiedener Form angewendet werden, z.B. als Folien, Fasern, Bändchen, Profile oder als Bindemittel für Lacke, Klebstoffe oder Kitte.

Die bisher bekannten polymeren Stabilisatoren sind mit dem Mangel behaftet, daß sie nicht in allen wichtigen anwendungstechnischen Parametern, zu welchen neben der Wirksamkeit noch die Flüchtigkeit, Migrationsfestigkeit (gleichbedeutend mit geringer Auswaschbarkeit) und Thermostabilität zählen, den technischen Erfordernissen genügen. Demgegenüber erfüllen die neuen, erfindungsgemäßen Stabilisatoren diese Anforderungen in hervorragender Weise. Sie besitzen eine sehr gute Stabilisatorwirksamkeit und sind weitgehend frei von auf physikalischen Eigenschaften beruhenden Nachteilen.

Die nachfolgenden Beispiele dienen der weiteren Erläuterung des Erfindungsgegenstandes.

**Beispiel 1**

2,2,7,7,9,9-Hexamethyl-1-oxa-3-(2,3-epoxypropyl)-3,8-diaza-4-oxo-spiro-(4,5)-decan und daraus erhaltenes Oligomer

Zu 150 $cm^3$ Toluol wurden nacheinander 24,0 g (0,1 mol) 2,2,7,7,9,9-Hexamethyl-1-oxa-3,8-diaza-4-oxo-spiro-(4,5)-decan, 18,5 g (0,2 mol) Epichlorhydrin, 5 Tropfen Tricaprylmethylammoniumchlorid ((R)Aliquat 336 der Fa. Fluka) und 40 g 50%ige Natronlauge (≙ 0,5 mol NaOH) gegeben, worauf man das Reaktionsgemisch 16 Stunden bei 65°C rührte. Nach dem Abstellen des Rührers bildeten sich zwei klare Phasen, welche getrennt wurden. Die organische Phase wurde dreimal mit 50 $cm^3$ Wasser gewaschen, über 50 g Natriumsulfat getrocknet, 30 min mit 1 g Aktivkohle bei Raumtemperatur verrührt und filtriert. Im Vakuum wurden die flüchtigen Anteile abgezogen. Es blieb ein farbloses Öl zurück, welches die in der Überschrift angegebene Epoxiverbindung ist. Sie wurde drei Stunden auf 170°C erwärmt und polymerisierte dabei zu einem festen, farblosen Harz vom Fp. 130 bis 184°C. Die Viskositätszahl (bestimmt in Anlehnung an DIN 53728 bei 25°C in einer 1 Gew.-%igen Lösung in Toluol) betrug 0,03.

**Beispiele 2 bis 12**

Es wurde wie in Beispiel 1 angegeben gearbeitet. In der nachstehenden Tabelle sind die Versuchsbedingungen sowie Angaben über die monomeren und polymeren Verfahrensprodukte zusammengestellt. Spalte 2 ("Verbindung Nr.") verweist auf die Aufstellung typischer monomerer Verfahrensprodukte in der Beschreibung Seiten 5/6, woraus sich auch das jeweils eingesetzte Polyalkyldiazaspirodecan ergibt.

Tabelle 1

| Bsp. Nr. | Verb. Nr. | Herstellung der Epoxiverbindung | | Fp. der Epoxiverbindung (°C) | Polymerisation | | Polymerisat | |
|---|---|---|---|---|---|---|---|---|
| | | Zeit (h) | Temp. (°C) | | Zeit (h) | Temp. (°C) | Fp. (°C) | Viskositätszahl 1) |
| 2 | 2 | 15 | 60 | 175 | 6 | 170 | 142-183 | 0,02 |
| 3 | 15 | 13 | 55 | 95-99 | 5 | 170 | 168-229 | 0,02 |
| 4 | 1 | 16 | 65 | Öl | 6 | 170 | 151-208 | 0,04 |
| 5 | 3 | 18 | 50 | 165 | 6 | 170 | 55-191 | 0,03 |
| 6 | 5 | 15 | 60 | Öl | 6 | 180 | 128-149 | 0,04 |
| 7 | 6 | 14 | 40 | Öl | 3 | 150 | 57-94 | 0,03 |
| 8 | 7 | 14 | 60 | Öl | 6 | 180 | 144-170 | 0,03 |
| 9 | 10 | 15 | 60 | Öl | 3 | 170 | 138-190 | 0,01 |
| 10 | 10 | 15 | 60 | Öl | 6 | 170 | 172-247 | 0,03 |
| 11 | 11 | 6 | 50 | 138 | 3 | 170 | 101-163 | 0,02 |
| 12 | 11 | 12 | 60 | Öl | 6 | 170 | 129-177 | 0,03 |

1) bestimmt in Anlehnung an DIN 53728 (1 Gew.-% in Toluol, 25°C)

Die folgenden Beispiele belegen die Überlegenheit der neuen Verbindungen gegenüber dem Stand der Technik.

**Beispiel 13**

| | |
|---|---|
| Zu 100,00 Gew.T. | Polypropylenpulver (MFI 230/5: 2-5 g/10 min) wurde unter Rühren eine Mischung (in Aceton) von |
| 0,20 Gew.T. | Calciumstearat, |
| 0,15 Gew.T. | Bis-[3,3-bis-(4′-hydroxi-3′tert.-butylphenyl)-butansäure]-glykolester, |
| 0,05 Gew.T. | Dioctadecyldisulfid, |
| 65,00 Gew.T. | Talkum (Typ OOS der Firma Lussenac) und |
| 0,50 Gew.T. | des zu prüfenden Stabilisators |

gegeben. Am Rotationsverdampfer wurde das Lösemittel entfernt und die Mischung mittels eines Laborextruders (Kurzkompressionsschnecke, Schneckendurchmesser: 20 mm, Länge 20 D, Düse 30 mm lang, 2 mm Durchmesser; Drehzahl: 125 UpM) extrudiert. Das Granulat wurde auf einer Windsor-Spritzgußmaschine der Type SP 50 zu 60 x 60 x 1 mm Platten verspritzt. Aus diesen Platten wurden T-förmige Prüfkörper ausgestanzt.

Zur Bestimmung der Wärmealterungsbeständigkeit wurden diese Prüfkörper in einem Umlufttrockenschrank in ein motorgetriebenes Gestell mit rotierenden Horden eingehängt und bei gleichmäßiger Frischluftzufuhr einer Temperaturbelastung von 140°C unterworfen.

Die Zeit, nach der an einigen Stellen eine beginnende lokale Versprödung auftrat, nach DIN 53 383 gekennzeichnet durch Bildung verfärbter, trüber, teilweise abbröckelnder Stellen, wurde festgehalten.

Die Ergebnisse zeigt Tabelle 2:

Tabelle 2

| Stabilisator gemäß Beispiel | beginnende Versprödung nach ... Tagen |
|---|---|
| 12 | 87 |
| Vergleich A[1)] | 65 |

1) (R)Chimasorb 944 gemäß DE 2636144

**Beispiel 14**

| | |
|---|---|
| Zu 100,00 Gew.T. | Polyethylenpulver (MFI 190/2,16: 2-5 g/10 min) wurde eine Lösung in Aceton von |
| 0,2 Gew.T. | des zu prüfenden Stabilisators gegeben. |

Aus dieser Mischung wurden wie in Beispiel 13 1 mm starke Platten hergestellt.

Zur Bestimmung der Wärmealterungsbeständigkeit wurden diese Platten in einem Trockenschrank einer Temperaturbelastung von 100°C unterworfen.

Nach 4 Wochen wurde die Vergilbung der Platten als Yellowness Index nach ASTM D 1925-70 gemessen (Hunterlab Colorimeter Modell D 25 M-2).

Tabelle 3

| Stabilisator gemäß Beispiel | Yellowness-Index (YI) | | |
|---|---|---|---|
| | unbehandelt | nach 4 Wochen | Änderung |
| 12 | 19,8 | 22,3 | 2,5 |
| Vergleich A | 20,6 | 26,9 | 6,3 |
| Vergleich C[2)] | 20,4 | 21,3 | 0,9 |

2) ohne zu prüfenden Stabilisator

**Beispiel 15**

| | |
|---|---|
| Zu 100,00 Gew.T. | Polyethylenpulver (Dichte 0,944 g/cm$^3$ MFI 190/2,16: 0,5 g/10 min) wurde eine Lösung in Aceton von |
| 0,2 Gew.T. | des zu prüfenden Stabilisators gegeben. |

Aus dieser Mischung wurden wie in Beispiel 13 1 mm starke Platten hergestellt.

Zur Bestimmung der Wärmealterungsbeständigkeit wurden diese Platten wie in Beispiel 14 behandelt und nach 4 Wochen wie in Beispiel 14 der Yellowness Index gemessen.

Tabelle 4

| Stabilisator gemäß Beispiel | Yellowness-Index (YI) | | |
|---|---|---|---|
| | unbehandelt | nach 4 Wochen | Änderung |
| 12 | 19,9 | 33,9 | 14,0 |
| Vergleich A | 29,2 | 87,0 | 57,8 |
| Vergleich C | 23,4 | 24,5 | 1,1 |

**Beispiel 16**

Zu 100,00 Gew.T. Polypropylenpulver (MFI 230/5: 2-5 g/10 min) wurde eine Lösung in Aceton von
0,10 Gew.T. Calciumstearat,
0,05 Gew.T. Bis-3,3-bis-(4′-hydroxi-3′tert.butylphenyl)-butansäureglykolester,
0,10 Gew.T. Tris-(2,4-di-tert.-butylphenyl)-phosphit und
0,50 Gew.T. des zu prüfenden Stabilisators gegeben.

Wie in Beispiel 13 wurde aus dieser Mischung ein Granulat hergestellt. Aus diesem Granulat wurden wie in Beispiel 13 1 mm starke Platten hergestellt.

Zur Bestimmung der Lichtstabilität wurden die Proben in einem "Suntest-Gerät" einer Dauerbestrahlung unterworfen (Gerät "Suntest" Fa. Heraeus POH, UV-Licht Filterkombination "UV-Spezialglas mit IR-reflektierender Schicht". Schwarztafeltemperatur 55°C ± 5°C. Keine Befeuchtung, kein Regen; Belichtungsabstand 32 cm).

Die Belichtung erfolgte bis zur "starken Oberflächenversprödung" (visuell).

Tabelle 5

| Stabilisator gemäß Beispiel | Belichtungszeit bis zur starken Rißbildung |
|---|---|
| 12 | > 3350 h |
| Vergleich A | < 1632 h |

**Beispiel 17**

Zur Bestimmung der Lichtstabilität wurden die in Beispiel 16 hergestellten Platten in einer Belichtungsapparatur der Firma Heraeus POH (Xenotest 1200) der Bestrahlung mit Wendelauf unterworfen. Die Strahlungsintensität wurde durch Spezialfilterglas d = 1,7 mm moduliert. Die Lichtbeständigkeit wurde nach DIN 53387 (102 min Trockenphase, 18 min Beregnen, Schwarztafeltemperatur 45°C, Luftfeuchtigkeit 70 %) gemessen. Die Belichtung erfolgte bis zur starken Oberflächenversprödung.

Tabelle 6

| Stabilisator gemäß Beispiel | Belichtungszeit bis zur starken Rißbildung |
|---|---|
| 12 | 2315 h |
| Vergleich A | 1885 h |

**Beispiel 18**

Zur Bestimmung der Lichtstabilität wurde ein wie in Beispiel 16 hergestelltes Granulat mit einer Laborfolienblasanlage zu 0,1 mm starken Folien verarbeitet. Daraus wurden Prüfkörper nach DIN 53455, Form 3, verkleinert im Maßstab 1:3, ausgestanzt. Diese Prüfkörper wurden unter den gleichen Bedingungen, wie in Beispiel 17 beschrieben, der

Bestrahlung mit Wendelauf unterworfen. Gemessen wurde die Belichtungszeit in Stunden und die Reißdehnung bestimmt. Die Reißdehnung wurde auf einer Zugprüfmaschine bei einer Abzugsgeschwindigkeit von 5 cm/min ermittelt.

Tabelle 7

| Stabilisator gemäß Beispiel | Belichtungszeit bis zum Erreichen des 50%-Werts der Ausgangsreißdehnung |
|---|---|
| 12 | 875 h |
| Vergleich A | 625 h |

**Beispiel 19**

| | |
|---|---|
| Zu 100,00 Gew.T. | Polypropylenpulver (MFI 230/5: 40-80 g/10 min) wurde eine Lösung in Aceton von |
| 0,5 Gew.T. | Pentaerythrityl-tetrakis-3-(3,5-di-tert.-butyl-4hydroxyphenyl)-propionat, |
| 0,10 Gew.T. | Tris-(2,4-di-tert.-butylphenyl)-phosphit und |
| 0,075 Gew.T. | des zu prüfenden Stabilisators gegeben. |

Aus dieser Mischung wurde wie in Beispiel 13 ein Granulat hergestellt.

Aus diesem Granulat wurde mit einer Laborfolienblasanlage eine 0,1 mm starke Folie hergestellt. Daraus wurden Prüfkörper von 50 x 35 mm hergestellt, die unter den gleichen Bedingungen, wie in Beipiel 17 beschrieben, der Bestrahlung mit Wendelauf unterworfen wurden. Zur Bestimmung der Lichtstabilität wurde bei den belichteten Proben mit einem Infrarotspektrographen gemäß DIN 53383/2 die

$$\text{Veränderung der Carbonylzahl} = \frac{E\ (1710\ cm^{-1})}{E\ (1260\ cm^{-1})}$$

gemessen. Stärkerer Anstieg der Carbonylzahl bedeutet höhere Oxidationsempfindlichkeit des Polypropylens und damit geringere Stabilität.

Tabelle 8

| Stabilisator gemäß Beispiel | Anstieg der Carbonylzahl nach 840 h |
|---|---|
| 12 | 0,3 |
| Vergleich B[3)] | 1,6 |

3) (R)Tinuvin 622 gemäß DE 2 719 131

**Beispiel 20**

| | |
|---|---|
| Auf 100,00 Gew.T. | Ethylen-Acrylsäure-Copolymergranulat (Type ESCN 5110 der Fa. Exxon Chemicals) wurden in einem Rollenbock |
| 0,1 Gew.T. | eines Verarbeitungsstabilisators, der unter der Bezeichnung[R] Hostanox VP ZnCS 1 im Handel ist, und |
| 0,2 Gew.T. | des zu prüfenden Stabilisators oberflächlich aufgetrommelt. |

Dieses Granulat wurde dann in einem Laborextruder zu Flachfolien (Stärke 180 µm, Breite 40 cm) extrudiert. Aus diesen Folien wurden Prüfkörper nach DIN 53 455, Form 3, verkleinert im Maßstab 1:3, ausgestanzt.

Diese Prüfkörper wurden unter den gleichen Bedingungen wie in Beispiel 17 beschrieben, der Bestrahlung mit Wendelauf unterworfen. Gemessen wurde die Belichtungszeit in Stunden und die Reißdehnung. Die Reißdehnung wurde auf einer Zugprüfmaschine bei einer Abzugsgeschwindigkeit von 5 cm/min ermittelt.

Tabelle 9

| Stabilisator gemäß Beispiel | Restreißdehnung nach 1800 h Belichtungszeit |
|---|---|
| 12 | 50 % |
| Vergleich A | 30 % |

## Patentansprüche

**Patentansprüche für folgende Vertragsstaaten : AT, BE, CH, DE, FR, GB, IT, LI, NL, SE**

**1.** Polymere Polyalkyl-1-oxa-diazaspirodecane der Formel I

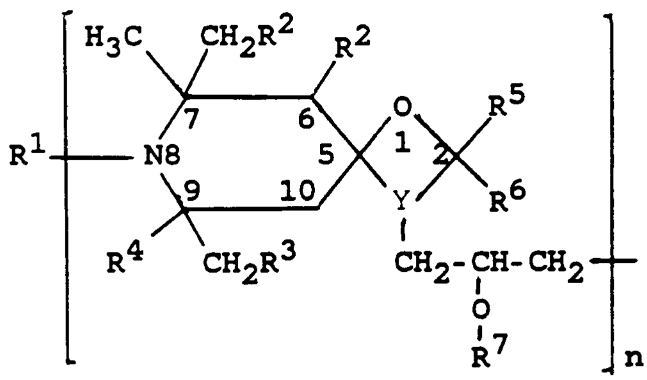

(I),

in welcher

n eine ganze Zahl von 2 bis 50 ist,

Y die Bedeutung einer Gruppe der Formel II oder III hat,

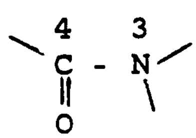

(II) ,

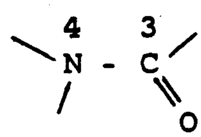

(III)

wobei die Indices 3 bzw. 4 die Ringpositionen im Diazaspirodecansystem angeben und eine Bindung des Stickstoffs mit einer $CH_2$-Gruppe der Propylen-2-oxygruppe verknüpft ist,

$R^1$ ein Wasserstoffatom, ein Sauerstoffatom, eine NO-Gruppe, eine $C_1$-$C_{12}$-Alkylgruppe, eine Allylgruppe, eine $C_1$-$C_{22}$-Acylgruppe oder eine Benzylgruppe bedeutet,

$R^2$ und $R^3$ entweder gleich sind und ein Wasserstoffatom oder eine $C_1$-$C_5$ Alkylgruppe bedeuten, wobei dann

$R^4$ eine Methylgruppe ist, oder

$R^2$ ein Wasserstoffatom oder eine $C_1$-$C_5$-Alkylgruppe ist und

$R^3$ und $R^4$ zusammen mit den sie verbindenden Kohlenstoffatomen eine $C_5$- oder $C_6$-Cycloalkylgruppe oder eine Gruppe der Formel

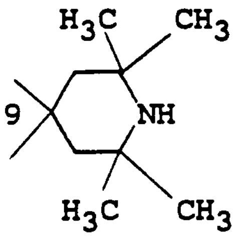

bilden,

$R^5$ und $R^6$ gleich oder verschieden sind und für ein Wasserstoffatom, eine $C_1$-$C_{30}$-Alkylgruppe, für eine unsubstituierte oder durch Chlor oder $C_1$-$C_4$-Alkyl substituierte Phenyl- oder Naphthylgruppe oder für eine unsubstituierte oder durch $C_1$-$C_4$-Alkyl substituierte $C_7$-$C_{12}$-Phenylalkylgruppe stehen, oder

$R^5$ und $R^6$ zusammen mit dem sie verbindenden Kohlenstoffatom eine unsubstituierte oder durch bis zu vier $C_1$-$C_4$-Alkylgruppen substituierte $C_5$-$C_{18}$-Cycloalkylgruppe oder eine Gruppe der Formel

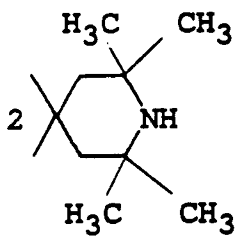

bilden, und

$R^7$ ein Wasserstoffatom oder eine $C_1$-$C_{22}$-Acylgruppe ist, oder

$R^7$ in der endständigen Monomereneinheit keine Bedeutung hat, so daß das Sauerstoffatom mit der endständigen $CH_2$-Gruppe verbunden ist und einen Oxiranring bildet.

2. Verwendung der Verbindungen nach Anspruch 1 zum Stabilisieren von synthetischen Polymeren.

3. Verwendung nach Anspruch 2, dadurch gekennzeichnet, daß das Polymere ein Polyolefin ist.

4. Verwendung nach Anspruch 2, dadurch gekennzeichnet, daß das Polymere ein halogenhaltiges Polymeres ist.

5. Verwendung nach Anspruch 2, dadurch gekennzeichnet, daß das Polymere ein Polyacrylat oder Polymethacrylat ist.

6. Verwendung nach Anspruch 2, dadurch gekennzeichnet, daß das Polymere ein Homo- oder Copolymeres von Styrol ist.

7. Verfahren zum Stabilisieren von synthetischen Polymeren gegen den schädigenden Einfluß von Licht, dadurch gekennzeichnet, daß man den Polymeren, gegebenenfalls neben bisher bekannten, stabilisierend wirkenden Stoffen, 0,01 bis 10 Gewichtsteile, bezogen auf Polymeres, eines Stabilisators nach Anspruch 1 zusetzt.

8. Gegen UV-Zersetzung stabilisierte synthetische Polymere, in denen 0,01 bis 10 Gewichtsteile, bezogen auf Polymeres, eines Stabilisators nach Anspruch 1 enthalten sind.

**Patentansprüche für folgenden Vertragsstaat : ES**

1. Verfahren zur Herstellung von polymeren Polyalkyl-1-oxa-diazaspirodecanen der Formel I

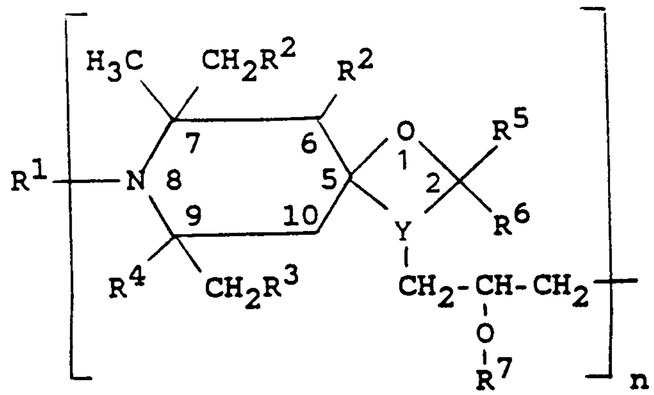

(I),

in welcher

n — eine ganze Zahl von 2 bis 50 ist,

y — die Bedeutung einer Gruppe der Formel II oder III hat,

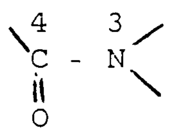

(II)

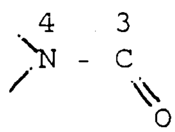

(III)

wobei die Indices 3 bzw. 4 die Ringpositionen im Diazaspirodecansystem angeben und eine Bindung des Stickstoffs mit einer $CH_2$-Gruppe der Propylen-2-oxygruppe verknüpft ist,

$R^1$ — ein Wasserstoffatom, ein Sauerstoffatom, eine NO-Gruppe, eine $C_1$-$C_{12}$-Alkylgruppe, eine Allylgruppe, eine $C_1$-$C_{22}$-Acylgruppe oder eine Benzylgruppe bedeutet,

$R^2$ und $R^3$ — entweder gleich sind und ein Wasserstoffatom oder eine $C_1$-$C_5$ Alkylgruppe bedeuten, wobei dann

$R^4$ — eine Methylgruppe ist, oder

$R^2$ — ein Wasserstoffatom oder eine $C_1$-$C_5$-Alkylgruppe ist und

$R^3$ und $R^4$ — zusammen mit den sie verbindenden Kohlenstoffatomen eine $C_5$- oder $C_6$-Cycloalkylgruppe oder eine Gruppe der Formel

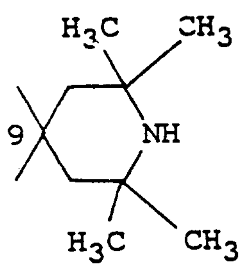

bilden,

$R^5$ und $R^6$ — gleich oder verschieden sind und für ein Wasserstoffatom, eine $C_1$-$C_{30}$-Alkylgruppe, für eine unsubstituierte oder durch Chlor oder $C_1$-$C_4$-Alkyl substituierte Phenyl- oder Naphthylgruppe oder für eine unsubstituierte oder durch $C_1$-$C_4$-Alkyl substituierte $C_7$-$C_{12}$-Phenylalkylgruppe stehen, oder

$R^5$ und $R^6$ — zusammen mit dem sie verbindenden Kohlenstoffatom eine unsubstituierte oder durch bis zu vier $C_1$-$C_4$-Alkylgruppen substituierte $C_5$-$C_{18}$-Cycloalkylgruppe oder eine Gruppe der For-

mel

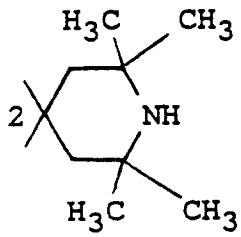

bilden, und

$R^7$ ein Wasserstoffatom oder eine $C_1$-$C_{22}$-Acylgruppe ist, oder

$R^7$ in der endständigen Monomereneinheit keine Bedeutung hat, so daß das Sauerstoffatom mit der endständigen $CH_2$-Gruppe verbunden ist und einen Oxiranring bildet, dadurch gekennzeichnet, daß man Verbindungen der Formel IV

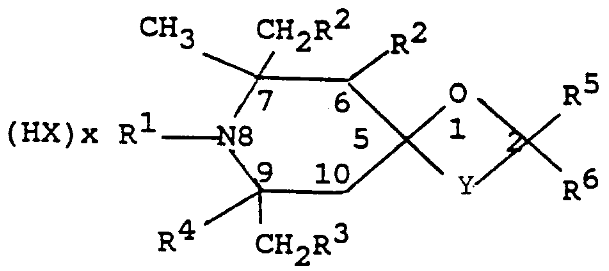

( IV ),

worin $R^1$, $R^2$, $R^3$, $R^4$, $R^5$, $R^6$ und Y die oben angegebene Bedeutung haben, mit Verbindungen der Formel V

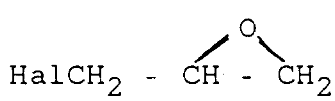

(V),

worin Hal ein Chlor, Brom- oder Jodatom ist, im Molverhältnis 1:1 bis 1:5 in einem inerten organischen Lösemittel in Anwesenheit der äquimolaren bis zwanzigfachen molaren Menge festen Alkalimetallhydroxids oder der entsprechenden Menge einer 20- bis 50 %igen wäßrigen Lösung eines solchen und 0,1 bis 5 Gew.-%, bezogen auf Verbindung (IV), eines Phasentransferkatalysators bei einer Temperatur von 20 bis 120°C zu Verbindungen der Formel VI

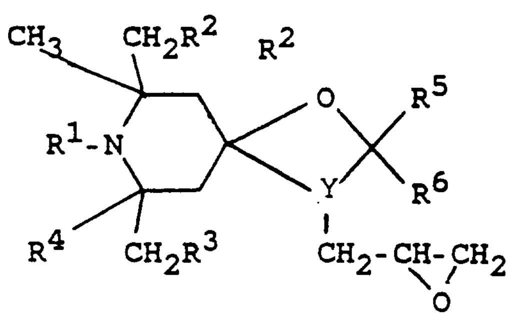

(VI),

worin $R^1$, $R^2$, $R^3$, $R^4$, $R^5$, $R^6$ und Y die obengenannte Bedeutung haben, umsetzt und die Verbindungen der Formel VI auf eine Temperatur von 100 bis 240°C erhitzt.

2. Verwendung der nach Anspruch 1 hergestellten Verbindungen zum Stabilisieren von synthetischen Polymeren.

3. Verwendung nach Anspruch 2, dadurch gekennzeichnet, daß das Polymere ein Polyolefin ist.

4. Verwendung nach Anspruch 2, dadurch gekennzeichnet, daß das Polymere ein halogenhaltiges Polymeres ist.

5. Verwendung nach Anspruch 2, dadurch gekennzeichnet, daß das Polymere ein Polyacrylat oder Polymethacrylat ist.

6. Verwendung nach Anspruch 2, dadurch gekennzeichnet, daß das Polymere ein Homo- oder Copolymeres von Polystyrol ist.

7. Verfahren zum Stabilisieren von synthetischen Polymeren gegen den schädigenden Einfluß von Licht, dadurch gekennzeichnet, daß man den Polymeren, gegebenenfalls neben bisher bekannten, stabilisierend wirkenden Stoffen, 0,01 bis 10 Gewichtsteile, bezogen auf Polymeres, eines nach Anspruch 1 hergestellten Stabilisators zusetzt.

8. Gegen UV-Zersetzung stabilisierte synthetische Polymere, in denen 0,01 bis 10 Gewichtsteile, bezogen auf Polymeres, eines nach Anspruch 1 hergestellten Stabilisators enthalten sind.

## Claims

**Claims for the following Contracting States : AT, BE, CH, DE, FR, GB, IT, LI, NL, SE**

1. A polymeric polyalkyl-1-oxa-diazaspirodecane of the formula I

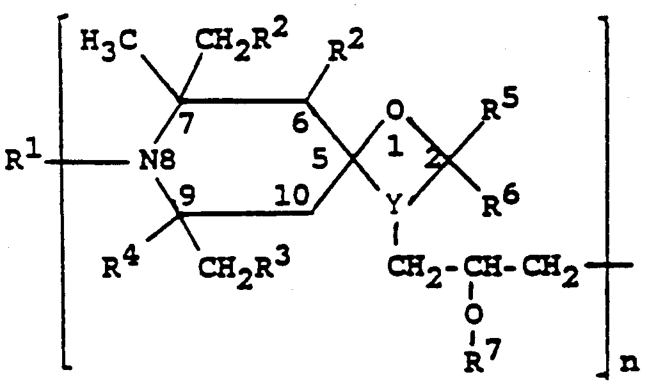

(I)

in which

| | |
|---|---|
| n | is an integer from 2 to 50, |
| Y | is a group of the formula II or III, |

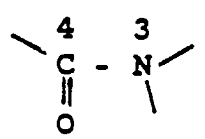 (II) 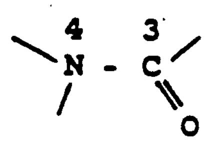 (III)

| | |
|---|---|
| | the indices 3 and 4 giving the ring positions in the diazaspirodecane system and one bond of the nitrogen being linked with a $CH_2$ group of the propylene-2-oxy group, |
| $R^1$ | is a hydrogen atom, an oxygen atom, an NO group, a $C_1$-$C_{12}$-alkyl group, an allyl group, a $C_1$-$C_{22}$-acyl group or a benzyl group, |
| $R^2$ and $R^3$ | are either identical and are a hydrogen atom or a $C_1$-$C_5$-alkyl group, |
| $R^4$ | then being a methyl group, or |
| $R^2$ | is a hydrogen atom or a $C_1$-$C_5$-alkyl group and |
| $R^3$ and $R^4$, | together with the carbon atoms linking them, form a $C_5$- or $C_6$-cycloalkyl group or a group of the formula |

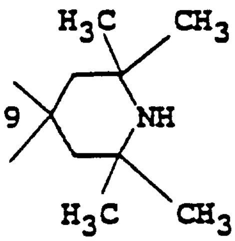

| | |
|---|---|
| $R^5$ and $R^6$ | are identical or different and represent a hydrogen atom, a $C_1$-$C_{30}$-alkyl group, represent an unsubstituted or chlorine- or $C_1$-$C_4$-alkyl-substituted phenyl or naphthyl group or represent an unsubstituted or $C_1$-$C_4$-alkyl-substituted $C_7$-$C_{12}$-phenylalkyl group, or |
| $R^5$ and $R^6$, | together with the carbon atom linking them, form an unsubstituted or up to tetra-$C_1$-$C_4$-alkyl-substituted $C_5$-$C_{18}$-cycloalkyl group or a group of the formula |

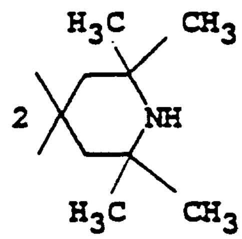

and

| | |
|---|---|
| $R^7$ | is a hydrogen atom or a $C_1$-$C_{22}$-acyl group, or |
| $R^7$, | in the terminal monomer unit, has no meaning so that the oxygen atom is linked with the terminal $CH_2$ group and forms an oxirane ring. |

**2.** The use of compounds as claimed in claim 1 to stabilize synthetic polymers.

**3.** The use as claimed in claim 2, wherein the polymer is a polyolefin.

**4.** The use as claimed in claim 2, wherein the polymer is a halogen-containing polymer.

**5.** The use as claimed in claim 2, wherein the polymer is a polyacrylate or polymethacrylate.

**6.** The use as claimed in claim 2, wherein the polymer is a styrene homo- or copolymer.

**7.** A process for stabilizing synthetic polymers against the harmful effect of light, which comprises adding to the polymers, optionally in addition to prior art stabilizing substances, 0.01 to 10 parts by weight, relative to the polymer, of a stabilizer as claimed in claim 1.

**8.** A synthetic polymer which has been stabilized against UV degradation and which contains 0.01 to 10 parts by weight, relative to the polymer, of a stabilizer as claimed in claim 1.

**Claims for the following Contracting State : ES**

1. A process for the preparation of a polymeric polyalkyl-1-oxa-diazaspirodecane of the formula I

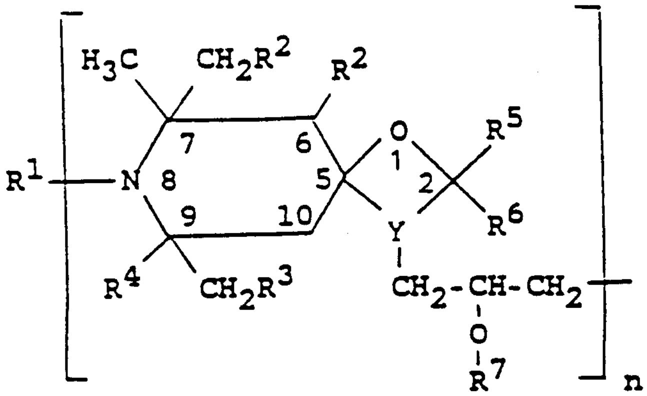

(I)

in which

| | |
|---|---|
| n | is an integer from 2 to 50, |
| Y | is a group of the formula II or III, |

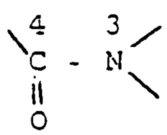

(II)

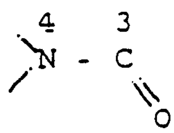

(III)

| | |
|---|---|
| | the indices 3 and 4 giving the ring positions in the diazaspirodecane system and one bond of the nitrogen being linked with a $CH_2$ group of the propylene-2-oxy group, |
| $R^1$ | is a hydrogen atom, an oxygen atom, an NO group, a $C_1$-$C_{12}$-alkyl group, an allyl group, a $C_1$-$C_{22}$-acyl group or a benzyl group, |
| $R^2$ and $R^3$ | are either identical and are a hydrogen atom or a $C_1$-$C_5$-alkyl group, |
| $R^4$ | then being a methyl group, or |
| $R^2$ | is a hydrogen atom or a $C_1$-$C_5$-alkyl group and |
| $R^3$ and $R^4$, | together with the carbon atoms linking them, form a $C_5$- or $C_6$-cycloalkyl group or a group of the formula |

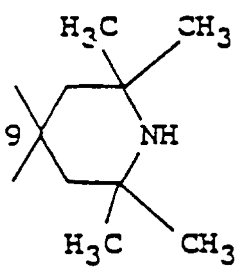

| | |
|---|---|
| $R^5$ and $R^6$ | are identical or different and represent a hydrogen atom, a $C_1$-$C_{30}$-alkyl group, represent an unsubstituted or chlorine- or $C_1$-$C_4$-alkyl-substituted phenyl or naphthyl group or represent an unsubstituted or $C_1$-$C_4$-alkyl-substituted $C_7$-$C_{12}$-phenylalkyl group, or |
| $R^5$ and $R^6$, | together with the carbon atom linking them, form an unsubstituted or up to tetra-$C_1$-$C_4$-alkyl-substituted $C_5$-$C_{18}$-cycloalkyl group or a group of the formula |

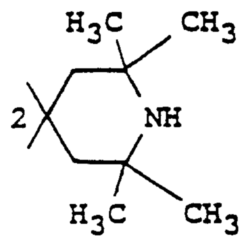

and

$R^7$ is a hydrogen atom or a $C_1$-$C_{22}$-acyl group, or

$R^7$, in the terminal monomer unit, has no meaning so that the oxygen atom is linked with the terminal $CH_2$ group and forms an oxirane ring, which comprises reacting compounds of the formula IV

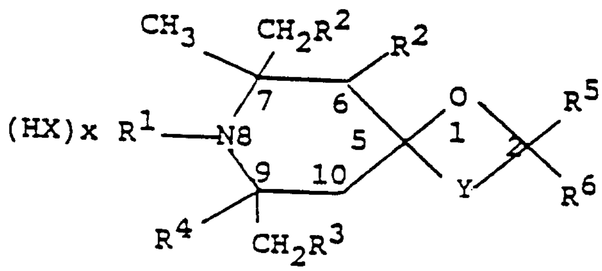

( IV ),

in which $R^1$, $R^2$, $R^3$, $R^4$, $R^5$, $R^6$ and Y have the meaning given above, with compounds of the formula V

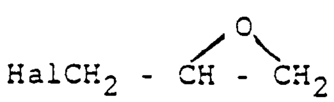

(V),

in which Hal is a chlorine, bromine or iodine atom, in the molar ratio 1:1 to 1:5 in an inert organic solvent in the presence of the equimolar to twenty-fold molar amount of solid alkali metal hydroxide or the corresponding amount of 20 to 50% strength aqueous solution of such an alkali metal hydroxide and 0.1 to 5% by weight, relative to the compound IV, of a phase transfer catalyst at a temperature of 20 to 120°C to give compounds of the formula VI

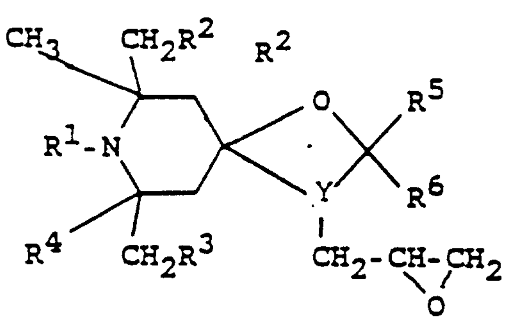

(VI),

in which $R^1$, $R^3$, $R^3$, $R^4$, $R^5$, $R^6$ and Y have the abovementioned meaning, and heating the compounds of the formula VI to a temperature of 100 to 240°C.

**2.** The use of compounds prepared as claimed in claim 1 to stabilize synthetic polymers.

**3.** The use as claimed in claim 2, wherein the polymer is a polyolefin.

4. The use as claimed in claim 2, wherein the polymer is a halogen-containing polymer.

5. The use as claimed in claim 2, wherein the polymer is a polyacrylate or polymethacrylate.

6. The use as claimed in claim 2, wherein the polymer is a styrene homo- or copolymer.

7. A process for stabilizing synthetic polymers against the harmful effect of light, which comprises adding to the polymers, optionally in addition to prior art stabilizing substances, 0.01 to 10 parts by weight, relative to the polymer, of a stabilizer prepared as claimed in claim 1.

8. A synthetic polymer which has been stabilized against UV degradation and which contains 0.01 to 10 parts by weight, relative to the polymer, of a stabilizer prepared as claimed in claim 1.

**Revendications**

**Revendications pour les Etats contractants suivants : AT, BE, CH, DE, FR, GB, IT, LI, NL, SE**

1. Polyalkyl-1-oxa-diazaspirodécanes polymères de formule I :

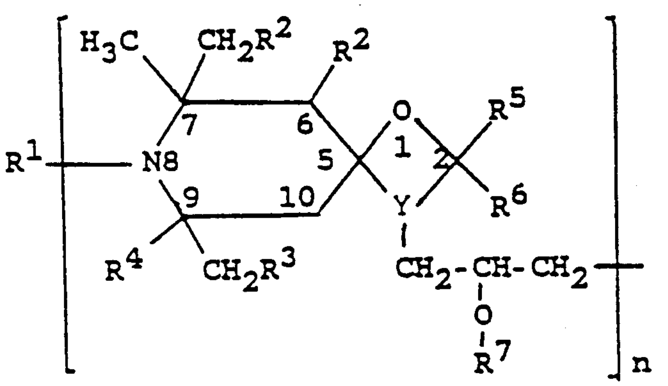

(I),

dans laquelle

n — est un nombre entier de 2 à 50,
Y — a la signification d'un groupe de formule II ou III

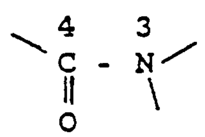

(II)

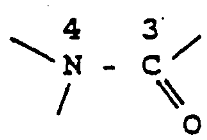

(III)

les indices 3 respectivement 4, indiquant les positions sur le cycle dans le système de diazaspirodécane et une liaison d'azote est liée à un groupe $CH_2$ du groupe propylène-2-oxy,

$R^1$ — représente un atome d'hydrogène, un atome d'oxygène, un groupe NO, un groupe alkyle en $C_1$-$C_{12}$, un groupe allyle, un groupe acyle en $C_1$-$C_{22}$ ou un groupe benzyle,
$R^2$ et $R^3$ — sont, soit identiques et représentent un atome d'hydrogène ou un groupe alkyle en $C_1$-$C_5$,
$R^4$ — étant alors un groupe méthyle, soit
$R^2$ — étant un atome d'hydrogène ou un groupe alkyle en $C_1$-$C_5$, et
$R^3$ et $R^4$, — ensemble avec les atomes de carbone qui les relient, forment un groupe cycloalkyle en $C_5$ ou $C_6$ ou un groupe de formule

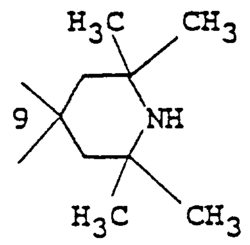

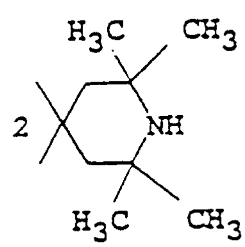

et

$R^7$ est un atome d'hydrogène ou un groupe acyle en $C_1$-$C_{22}$ ou

$R^7$ dans le motif monomère terminal n'a pas de signification, de sorte que l'atome d'oxygène est relié au le groupe $CH_2$ terminal et forme un cycle oxirane.

2. Utilisation des composés préparés selon la revendication 1, pour stabiliser des polymères synthétiques.

3. Utilisation selon la revendication 2, caractérisée en ce que le polymère est une polyoléfine.

4. Utilisation selon la revendication 2, caractérisée en ce que le polymère est un polymère halogéné.

5. Utilisation selon la revendication 2, caractérisée en ce que le polymère est un polyacrylate ou un polyméthacrylate.

6. Utilisation selon la revendication 2, caractérisée en ce que le polymère est un homo- ou copolymère du styrène.

7. Procédé pour stabiliser des polymères synthétiques contre la dégradation induite par la lumière, caractérisé en ce que l'on ajoute aux polymères, éventuellement au côté de substances connues jusqu'à présent ayant une action stabilisante, de 0,01 à 10 parties en poids, par rapport aux polymère, d'un stabilisant selon la revendication 1.

8. Polymères synthétiques stabilisés contre la dégradation par la lumière UV qui contiennent de 0,01 à 10 parties en poids par rapport aux polymères d'un stabilisant selon la revendication 1.

**Revendications pour l'Etat contractant suivant : ES**

1. Procédé pour la préparation de polyalkyl-1-oxa-diazaspirodécanes polymères de formule I :

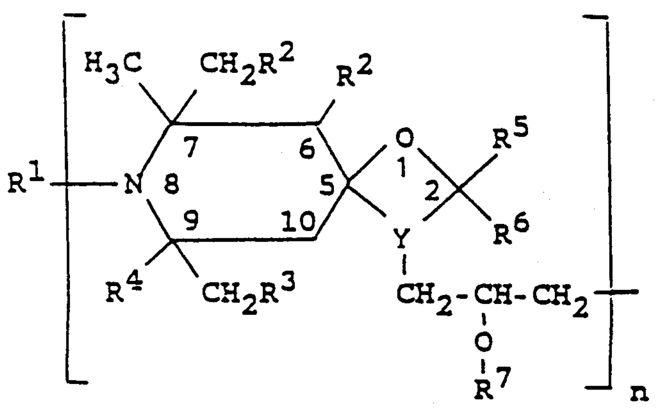

(I),

dans laquelle

n est un nombre entier de 2 à 50,

Y a la signification d'un groupe de formule II ou III

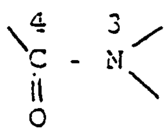

(II)

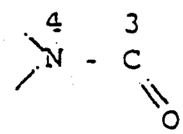

(III)

les indices 3, respectivement 4, indiquant les positions sur le cycle dans le système de diazaspirodécane et une liaison d'azote est liée à un groupe $CH_2$ du groupe propylène-2-oxy,

$R^1$ représente un atome d'hydrogène, un atome d'oxygène, un groupe NO, un groupe alkyle en $C_1$-$C_{12}$, un groupe allyle, un groupe acyle en $C_1$-$C_{22}$ ou un groupe benzyle,

$R^2$ et $R^3$ sont soit identiques et représentent un atome d'hydrogène ou un groupe alkyle en $C_1$-$C_5$,

$R^4$ étant alors un groupe méthyle, soit

$R^2$ étant un atome d'hydrogène ou un groupe alkyle en $C_1$-$C_5$, et

$R^3$ et $R^4$, ensemble avec les atomes de carbone qui les relient, forment un groupe cycloalkyle en $C_5$ ou $C_6$ ou un groupe de formule

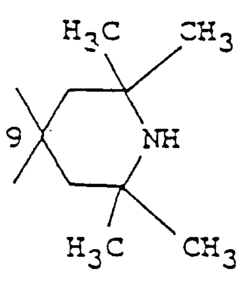

$R^5$ et $R^6$ sont identiques ou différents et représentent un atome d'hydrogène, un groupe alkyle en $C_1$-$C_{30}$, un groupe phényle ou naphtyle non substitué ou substitué par chlore ou alkyle en $C_1$-$C_4$ ou un groupe phénylalkyle en $C_7$-$C_{12}$ non substitué ou substitué par alkyle en $C_1$-$C_4$, ou

$R^5$ et $R^6$, ensemble avec l'atome de carbone qui les relie, représentent un groupe cycloalkyle en $C_5$-$C_{18}$ non substitué ou substitué par 1 à 4 groupes alkyle en $C_1$-$C_4$ ou forment un groupe de formule

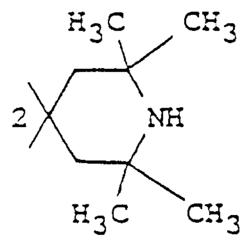

et

$R^7$ est un atome d'hydrogène ou un groupe acyle en $C_1$-$C_{22}$ ou

$R^7$ dans le motif monomère terminal n'a pas de signification, de sorte que l'atome d'oxygène est relié aux groupe $CH_2$ terminal et forme un cycle oxirane, caractérisé en ce que l'on fait réagir des composés de formule IV

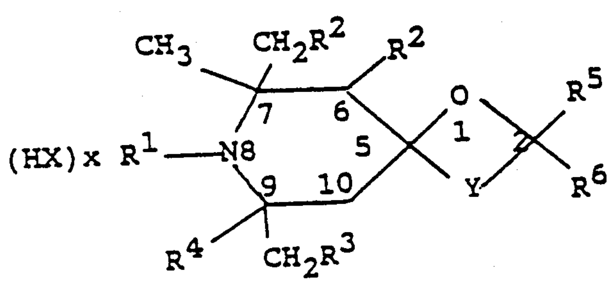

(IV),

dans laquelle $R^1$, $R^2$, $R^3$, $R^4$, $R^5$, $R^6$ et Y ont les significations données ci-dessus avec des composés de formule V

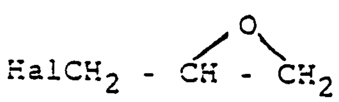

(V),

dans laquelle Hal est un atome de chlore, de brome ou d'iode, dans un rapport molaire de 1:1 à 1:5 dans un solvant organique inerte en présence de la quantité équimolaire jusqu'à 20 fois molaire d'hydroxyde de métaux alcalins solide, ou de la quantité correspondante d'une solution aqueuse d'un hydroxyde à 20 à 50 % et de 0,1 à 5 % en poids par rapport au composé (IV) d'un catalyseur de transfert de phases, à une température de 20 à 120 °C, pour obtenir le composé de formule (VI)

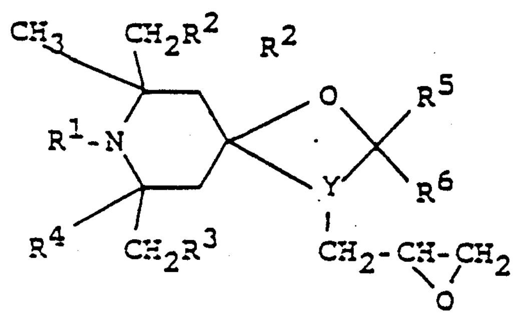

(VI),

dans laquelle $R^1$, $R^2$, $R^3$, $R^4$, $R^5$, $R^6$ et Y ont les significations données ci-dessus, et on chauffe les composés de formule VI à une température de 100 à 240 °C.

2. Utilisation des composés préparés selon la revendication 1 pour stabiliser des polymères synthétiques.

3. Utilisation selon la revendication 2, caractérisée en ce que le polymère est une polyoléfine.

4. Utilisation selon la revendication 2, caractérisée en ce que le polymère est un polymère halogéné.

5. Utilisation selon la revendication 2, caractérisée en ce que le polymère est un polyacrylate ou un polyméthacrylate.

6. Utilisation selon la revendication 2, caractérisée en ce que le polymère est un homo- ou copolymère du polystyrène.

7. Procédé pour stabiliser des polymères synthétiques contre la dégradation induite par la lumière, caractérisé en ce que l'on ajoute aux polymères éventuellement au côté des substances connues jusqu'à présent ayant une action stabilisante, de 0,01 à 10 parties en poids par rapport aux polymères d'un stabilisant préparé selon la revendication 1.

8. Polymères synthétiques stabilisés contre la dégradation par la lumière UV qui contiennent de 0,01 à 10 parties en poids par rapport aux polymères d'un stabilisant selon la revendication 1.